# FASCICULE DE BREVET EUROPEEN

(11) **EP 2 238 104 B1**
(45) Date de publication et mention de la délivrance du brevet: **14.12.2016**
(21) Numéro de dépôt: 09704625.4
(22) Date de dépôt: 12.01.2009
(51) Int. Cl.: C07C 255/04, B01J 31/02, B01J 31/18, B01J 31/14

(54) **PROCEDE DE FABRICATION DE COMPOSES COMPRENANT DES FONCTIONS NITRILES**
VERFAHREN ZUR HERSTELLUNG VON VERBINDUNGEN MIT NITRILFUNKTIONEN
PROCESS FOR PRODUCING COMPOUNDS COMPRISING NITRILE FUNCTIONS

(30) Priorité: 25.01.2008 FR 0800381
(43) Date de publication de la demande: 13.10.2010
(73) Titulaire: Invista Technologies S.à.r.l., 9000 St. Gallen (CH)
(72) Inventeur: MASTROIANNI, Sergio, 69006 Lyon (FR)
(74) Mandataire: Carpmaels & Ransford LLP
(86) Numéro de dépôt international: PCT/EP2009/050265
(87) Numéro de publication internationale: WO 2009/092639

(56) Documents cités:
- DE-A1- 10 314 761
- US-A- 3 864 380
- J. SERWATOWSKI ET AL: "New Tetrameric Alkylmetal Boryloxides of Zinc and Cadnium with Heterocubane structure" INORG. CHEM., vol. 38, no. 22, 1999, pages 4937-4941, XP002499912 cité dans la demande
- J. SERWATOWSKI ET AL: "Diverse Reactivity of Dialkylaluminium Dimesitylboryloxides. Synthetic and Structural Study" INORG. CHEM., vol. 39, no. 25, 2000, pages 5763-5767, XP002499913 cité dans la demande
- V. C. GIBSON: "Formation and Unexpected Catalytic Reactivity of Organoaluminium Boryloxides" INORG. CHEM., vol. 40, no. 5, 2001, pages 826-827, XP002499914 cité dans la demande

## Description

La présente invention concerne un procédé de fabrication de composés comprenant au moins une fonction nitrile par hydrocyanation d'un composé comprenant au moins une insaturation non conjuguée.

Elle se rapporte plus particulièrement à un procédé de fabrication mettant en oeuvre la réaction du cyanure d'hydrogène avec un composé organique comprenant une insaturation non conjuguée en présence d'un système catalytique comprenant un complexe du nickel à l'état d'oxydation zéro (appelé ci-après Ni(0)) avec au moins un ligand organophosphoré et un cocatalyseur appartenant à la famille des acides de Lewis.

De tels procédés sont connus depuis de nombreuses années et sont exploités industriellement notamment pour la production d'un grand intermédiaire chimique, l'adiponitrile. Ce composé est notamment utilisé dans la fabrication de l'hexaméthylène diamine qui est un monomère important pour la fabrication des polyamides et également un intermédiaire dans la synthèse des composés diisocyanate.

Ainsi, la société DU PONT DE NEMOURS a mis au point et exploité un procédé de fabrication de l'adiponitrile par double hydrocyanation du butadiène. Cette réaction est généralement catalysée par un système catalytique comprenant un complexe du nickel(0) avec des ligands organophosphorés. Ce système comprend également un cocatalyseur, notamment dans l'étape de seconde hydrocyanation, c'est-à-dire hydrocyanation de composés insaturés comprenant une fonction nitrile tels que les pentènenitriles en composés dinitriles.

De nombreux cocatalyseurs ont été proposés dans les brevets et sont généralement des composés appartenant à la famille des acides de Lewis. Le rôle de ce cocatalyseur ou promoteur est de limiter la production de sous-produits et donc de favoriser la formation de composés de dinitriles linéaires par rapport à la formation de dinitriles ramifiés.

Ainsi, de nombreux halogénures métalliques tels que le chlorure de zinc, le bromure de zinc, le chlorure stanneux, le bromure stanneux ont déjà été proposés, par exemple, dans le brevet US 3 496 217. Le chlorure de zinc est le cocatalyseur préféré.

Il a également été proposé des composés organiques du bore tels que le triphenyl de bore ou des composés comprenant deux atomes de bore tels que décrits dans les brevets US 3 864 380 et US 3 496 218, ou des composés organiques de l'étain comme dans le brevet US 4 874 884.

Ces cocatalyseurs ont des propriétés différentes et permettent d'obtenir des sélectivités en dinitriles linéaires tels que l'adiponitrile différentes. Certains de ces cocatalyseurs présentent des inconvénients liés à la difficulté à les extraire du milieu réactionnel ou à la possibilité et facilité d'extraire le système catalytique ou le ligand du nickel (0) en présence de ce cocatalyseur pour le recycler.

Il existe toujours un besoin de trouver de nouveaux cocatalyseurs permettant d'obtenir des sélectivités en dinitriles linéaires de niveaux acceptables et d'utilisation facile.

Un des buts de la présente invention est de proposer une nouvelle famille de cocatalyseurs compatibles et donnant des niveaux de sélectivité en adiponitrile convenables dans la réaction d'hydrocyanation des pentènitriles.

A cet effet, l'invention propose un procédé de fabrication de composés comprenant au moins une fonction nitrile par hydrocyanation d'un composé organique comprenant au moins une insaturation non conjuguée comprenant de 2 à 20 atomes de carbone par réaction avec le cyanure d'hydrogène en présence d'un système catalytique comprenant un complexe du nickel à l'état d'oxydation zéro avec au moins un ligand organophosphoré choisi dans le groupe comprenant les organophosphites, organophosphonites, organophosphinites et organosphosphines et un cocatalyseur caractérisé en ce que le cocatalyseur est un composé organométallique répondant à la formule générale I : 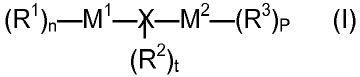 dans laquelle :
R¹, R², R³ identiques ou différents représentent un radical organique aliphatique ramifié ou non, un radical aromatique ou cycloaliphatique substitué ou non, ou un atome d'halogène,
M¹, M², identiques ou différents représentent un élément de valence m¹, m² respectivement appartenant choisi dans le groupe comprenant le zinc, le bore, l'aluminium, le cadmium, le gallium, l'indium et l'étain M¹ et M² ne pouvant représenter simultanément le bore,
X représente l'oxygène,
n est un nombre entier égal à m¹-1
p est un nombre entier égal à m²-1
t est un nombre entier égal à x-2.

Selon un mode de réalisation préféré, les éléments M¹ et M² sont choisis dans le groupe comprenant le bore, l'aluminium et le zinc.

De préférence, M² représente l'aluminium ou le zinc, M¹ représente le bore ou l'aluminium. Dans un mode de réalisation encore plus préféré, M² représente l'aluminium ou le zinc et M¹ représente le bore ou l'aluminium.

Selon une autre caractéristique de l'invention, les radicaux R¹ et R³ sont des radicaux alkyles linéaires ou ramifiés comprenant de 1 à 6 atomes de carbone, des radicaux phényls substitués ou non, ou des atomes d'halogène, de préférence le chlore.

Les composés pouvant être utilisés comme catalyseurs dans les procédés d'hydrocyanation sont, par exemple :

(C₂H₅)₂-B-O-Al-(C₂H₅)₂

(C₂H₅)₂-B-O-Al-Cl₂

(IBᵤ)₂-Al-O-Al-(iBᵤ)₂

(mes)₂-B-O-Al-(C₂H₅)₂

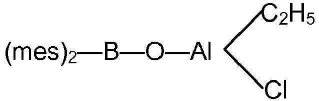

(mes)₂-B-O-Al-Cl₂

(mes)₂-B-O-Zn-C₂H₅

(C₂H₅)₂-Al-O-AI-(C₂H₅)₂

dans lesquelles
iBu représente le radical isobutyle
mes représente un groupement mésityl (2,4,6 triméthylphényl).

Les composés utilisés comme catalyseur, conformément à l'invention, peuvent être présents sous forme de molécules indépendantes ou sous forme agrégée par des liaisons non covalentes telles que des liaisons datives formant ainsi des dimères, trimères ou tétramères.

A titre d'exemple, le composé (mes)₂-B-O-Al-(C₂H₅)₂ est généralement présent sous forme dimère [(µ-mes)₂-B-O-Al-(C₂H₅)₂]₂. Pour plus de clarté, dans le présent texte il ne sera fait référence qu'à la molécule simple de chaque composé sans pour cela limiter la portée du brevet à cette forme simple.

Dans un mode préféré de l'invention, le système catalytique de l'invention contient un cocatalyseur conforme à l'invention dans un rapport molaire de cocatalyseur par rapport au nombre d'atomes de nickel compris entre 0.01 et 50 et de préférence entre 0.1 et 10.

Les cocatalyseurs de l'invention sont des composés décrits dans la littérature ainsi que leur procédé de fabrication. On peut citer, à titre d'exemple, comme articles décrivants les procédés de fabrication de ces composés les articles de J. Serwatowski et al. publiés dans Inorganic Chemistry, 1999, 38, 4937 et Inorganic Chemistry, 2000, 39, 5763 ainsi que l'article de V.G. Gibson et al. publié dans Inorganic Chemistry, 2001, 40 826.

Des exemples de fabrication de ces composés sont donnés ci-après.

Le système catalytique de l'invention comprend un complexe du nickel (0) avec au moins un composé organophosphoré, préférentiellement un composé monodentate tel que le triphénylphosphite ou le tritolylphosphite décrits par exemple, dans les brevets US3496215, DE19953058, FR1529134, FR2069411, US3631191, US3766231, FR2523974.ou un composé bidentate tel que les composés organophosphites décrits dans les brevets WO9906355, WO9906356, WO9906357, WO9906358, WO9952632, WO9965506, WO9962855, US5693843, WO961182, WO9622968, US5981772, WO0136429, WO9964155, WO0213964, US6127567

Il est également possible d'utiliser des complexes du nickel (0) avec des composés organophoshines monodentates ou bidentates tels que décrits dans les brevets WO02/30854, WO02/053527, WO03/068729, WO04/007435, WO04/007432, FR2845379, WO2004/060855 et plus particulièrement le DPPX décrit dans le brevet WO2003031392.

De même, le système catalytique de l'invention peut comprendre un complexe du nickel (0) avec des composés organophosphorés monodentates ou bidentates appartenant à la famille des organophosphonites ou organophosphinites..

Il est également possible d'utiliser les cocatalyseurs de l'invention avec un complexe du nickel (0) obtenu avec un mélange de ligand monodentate organophosphite et de ligand bidentate choisi dans les familles de composés appartenant aux organophosphites, organophosphonites, organophosphinites ou organophosphines tels que décrits dans les brevets WO03011457, WO2004/065352 .

La description du procédé d'hydrocyanation est réalisée dans plusieurs brevets dont ceux cités précédemment et également dans les articles de C.A. TOLMAN publiés dans les revues Organometallics 3 (1984) 33 et s, Advances in Catalysis (1985) 33-1 ; J. Chem. Soc. Chem. Commun (1991)-1292 et (1991)-803.

Brièvement, le procédé de fabrication de composés comprenant au moins une fonction nitrile et plus particulièrement de composés dinitriles tels que l'adiponitrile consiste à faire réagir dans une première étape une dioléfine telle que le 1, 3 butadiène avec du cyanure d'hydrogène, généralement en absence de solvant et en présence d'un système catalytique. La réaction est conduite sous pression pour être en milieu liquide. Les composés nitriles insaturés sont séparés par distillations successives. Les composés nitriles linéaires tels que les pentènenitriles sont alimentés dans une seconde étape d'hydrocyanation.

Avantageusement, les nitriles insaturés non linéaires obtenus en première étape, sont soumis à une étape d'isomérisation pour les transformer en nitriles insaturés linéaires qui sont introduits également dans la seconde étape d'hydrocyanation.

Dans la seconde étape d'hydrocyanation, les nitriles insaturés linéaires sont mis en réaction avec du cyanure d'hydrogène en présence d'un système catalytique.

Les composés dinitriles formés sont séparés par distillations successives après extraction du système catalytique du milieu réactionnel. Plusieurs procédés d'extraction du système catalytique sont décrits, par exemple, dans les brevets US3 773 809, 4 082 811, 4 339 395 et 5 847 191. Généralement, le système catalytique peut être séparé du milieu réactionnel par décantation en deux phases obtenue par le contrôle des rapports entre les composés mononitriles et les composés dinitriles contenus dans le milieu. Cette séparation peut être améliorée par addition d'ammoniac. Il est également possible de faire précipiter le système catalytique pour le récupérer et le recycler ou d'utiliser un solvant non polaire pour extraire le système catalytique et le séparer des produits nitriles.

### Les conditions de température de ces différentes étapes sont comprises entre 10 et 200°C

Les systèmes catalytiques utilisés dans les première et seconde étapes d'hydrocyanation ainsi que dans l'étape d'isomérisation sont généralement semblables, c'est-à-dire qu'ils contiennent un complexe de nickel (0) identique. Toutefois, le rapport entre le nombre d'atome de nickel et le nombre de molécules de ligand peut être différent dans chacune de ces étapes ainsi que la concentration du système catalytique dans le milieu.

Préférentiellement, le cocatalyseur est présent uniquement dans le système catalytique utilisé pour la seconde étape d'hydrocyanation. Toutefois, il peut être également présent dans l'étape d'isomérisation.

Les caractéristiques et performances du procédé et donc du système catalytique utilisé sont déterminées et illustrées par le taux de transformation (TT) du composé introduit, notamment du mononitrile insaturé introduit dans la seconde étape et par la linéarité en dinitriles linéaires produits, c'est-à-dire le nombre de mole de dinitriles linéaires par rapport au nombre de mole de dinitriles formés. Dans le cas de la fabrication de l'adiponitrile, la linéarité correspond au pourcentage de mole d'adiponitrile (AdN) obtenue par rapport aux nombres de moles de dinitriles formés (AdN + ESN + MGN).

L'invention sera mieux illustrée par les exemples donnés ci-dessous uniquement à titre indicatif, relatifs à la fabrication d'adiponitrile par hydrocyanation de 3-pentènenitrile. Dans ces exemples, le 3-pentènenitrile est un composé commercial.

Dans ces exemples, les abréviations suivantes sont utilisées :
- Cod: cyclooctadiène
- 3PN: 3-pentènenitrile
- AdN: Adiponitrile
- ESN: éthylsuccinonitrile
- MGN: méthylglutaronitrile
- TTP: tri-paratolylphosphite
- TEA: triéthylaluminium
- DEAC: diéthylaluminium chlorure
- EADC: éthylaluminium dichlorure
- TiBAO: tetraisobutyldialuminoxane
- mes: groupement mésityl (2,4,6 triméthylphényl)
- Et: groupement éthyle
- iBu: groupement isobutyle
- Ph: groupement phényle
- DPPX: bis(diphénylphosphinométhyl)-1,2-benzène
- TT(Y): taux de transformation du produit à hydrocyaner Y correspondant au rapport du nombre de moles transformées de Y sur le nombre de moles initiales de Y
- Linéarité (L): rapport du nombre de moles d'AdN formées sur le nombre de moles de dinitriles formées (somme des moles d'AdN, ESN et MGN)

Synthèse des composés cocatalyseurs de formule I Les synthèses des différents composés ont été réalisées selon les procédés décrits dans les articles publiés dans la revue Inorganic Chemistry cités précédemment. Ces synthèses sont réalisées sous atmosphère d'argon

### Exemple 1: Synthèse de (mes)₂-B-O-Al-(C₂H₅)₂

179 mg d'une solution molaire de TEA dans l'hexane sont ajoutés rapidement et sous agitation à une solution d'acide dimésitylborinique (68 mg) dans du toluène anhydre (1 ml). Le mélange est maintenu sous agitation et à température pendant 30 minutes avant d'être utilisé en totalité comme catalyseur dans le test d'hydrocyanation.

### Exemple 2:

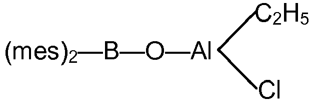

175 mg d'une solution molaire de DEAC dans l'hexane sont ajoutés rapidement et sous agitation à une solution d'acide dimésitylborinique (66 mg) dans du toluène anhydre (1 ml). Le mélange est maintenu sous agitation et à température pendant 30 minutes avant d'être utilisé en totalité pour le test d'hydrocyanation.

### Exemple 3: (mes)₂-B-O-Al-Cl₂

182 mg d'une solution molaire de EADC dans l'hexane sont ajoutés rapidement et sous agitation à une solution d'acide dimésitylborinique (68 mg) dans du toluène anhydre (1 ml). Le mélange est maintenu sous agitation et à température pendant 30 minutes avant d'être utilisé en totalité pour le test d'hydrocyanation.

### Exemple 4: (mes)₂-B-O-Zn-C₂H₅

221 mg d'une solution 1.1 molaire de ZnEt₂ dans le toluène sont ajoutés rapidement et sous agitation à une solution d'acide dimésitylborinique (67 mg) dans du toluène anhydre (1 ml). Le mélange est maintenu sous agitation et à température pendant 30 minutes avant d'être utilisé en totalité pour le test d'hydrocyanation.

### Exemples : Hydrocyanation du 3-PN en AdN

Le mode opératoire est le suivant :
Sous atmosphère d'argon, dans un tube de verre type Schott de 60 ml équipé d'un bouchon-septum, sont chargés successivement:
   - le ligand [5 équivalents molaires de ligand par atome de Ni si le ligand est un monodentate tel que le TTP ou 2,5 équivalents molaires de ligand par atome de Ni si le ligand est bidentate tel que la DPPX]
   - 1.21 g (15 mmol, 30 équivalents) de 3PN anhydre
   - 138 mg (0.5 mmol, 1 équivalent) de Ni(Cod)₂
   - acide de Lewis (voir les indications dans les tableaux I et II ci-dessous pour la nature et la quantité)

Le mélange est porté sous agitation à 70°C. La cyanhydrine de l'acétone, générateur de HCN, est injectée dans le milieu réactionnel par un pousse-seringue à un débit de 0,45 ml par heure. Après 3 heures d'injection le pousse-seringue est arrêté. Le mélange est refroidi à température ambiante, dilué avec de l'acétone et analysé par chromatographie en phase gazeuse.

Les résultats sont regroupés dans les tableaux I et II suivants

**Tableau I: exemples 5 à 7 comparatifs**

| exemple | ligand | Acide de Lewis | Acide de Lewis/Ni | TT (3PN) | Linéarité |
|---|---|---|---|---|---|
| 5 | TTP | (mes)₂BOH | 1 | 23.6 | 67.7 |
| 6 | DPPX | ZnCl2 | 1 | 72.8 | 59.0 |
| 7 | TTP | Ph₂BOBPh₂ | 0.5 | 14.3 | 73.8 |

**Tableau 2: exemples 8 à 16**

| exemple | ligand | Acide de Lewis | Acide de Lewis/Ni | TT (3PN) | Linéarité |
|---|---|---|---|---|---|
| 8 | TTP | TiBAO | 0.5 | 47.9 | 76.8 |
| 9 | TTP | TiBAO | 0.25 | 36.7 | 78.7 |
| 10 | TTP | Ex. 1 | 0.5 | 38.2 | 76.2 |
| 11 | TTP | Ex. 2 | 0.5 | 34.0 | 73.5 |
| 12 | TTP | Ex. 3 | 0.5 | 35.8 | 71.1 |
| 13 | TTP | Ex. 4 | 0.5 | 51 | 78.2 |
| 14 | DPPX | TiBAO | 0.5 | 78.4 | 84.7 |
| 15 | DPPX | TiBAO | 0.25 | 77.8 | 86.4 |
| 16 | DPPX | Ex. 4 | 0.5 | 33.3 | 84.1 |

## Revendications

1. Procédé de fabrication de composés comprenant au moins une fonction nitrile par hydrocyanation d'un composé organique comprenant au moins une insaturation non conjuguée comprenant de 2 à 20 atomes de carbone par réaction avec le cyanure d'hydrogène en présence d'un système catalytique comprenant un complexe du nickel à l'état d'oxydation zéro avec au moins un ligand organophosphoré choisi dans le groupe comprenant les organophosphites, organophosphonites, organophosphinites et organosphosphines et un cocatalyseur **caractérisé en ce que** le cocatalyseur est un composé organométallique répondant à la formule générale I : 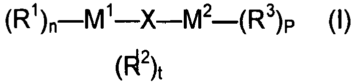 dans laquelle :
R¹, R², R³ identiques ou différents représentent un radical organique aliphatique ramifié ou non, un radical aromatique ou cycloaliphatique substitué ou non, ou un atome d'halogène,
M¹, M², identiques ou différents représentent un élément de valence m¹, m² respectivement choisi dans le groupe comprenant le zinc, le bore, l'aluminium, le cadmium, le gallium, l'indium et l'étain, M¹ et M² ne pouvant représenter simultanément le bore,
X représente l'oxygène
n est un nombre entier égal à m¹-1
p est un nombre entier égal à m²-1
t est un nombre entier égal à x-2.

2. Procédé selon la revendication 1, **caractérisé en ce que** M² représente l'aluminium ou le zinc, M¹ représente le bore ou l'aluminium.

3. Procédé selon la revendication 1, **caractérisé en ce que** M² représente l'aluminium ou le zinc, M¹ représente le bore ou l'aluminium

4. Procédé selon la revendication 1 à 3, **caractérisé en ce que** les radicaux R¹ et R³ représentent un radical alkyl comprenant de 1 à 6 atomes de carbone, un radical phényl substitué ou non ou un atome d'halogène.

5. Procédé selon l'une des revendications précédentes, **caractérisé en ce que** le cocatalyseur est choisi dans le groupe comprenant les composés suivants ou leurs dimères, trimères ou tetramères :
(C₂H₅)₂-B-O-Al-(C₂H₅)₂
(C₂H₅)₂-B-O-Al-Cl₂
(iBᵤ)₂-Al-O-Al-(iBᵤ)₂
(mes)₂-B-O-Al-(C₂H₅)₂ 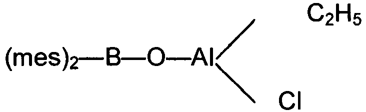
(mes)₂-B-O-Al-Cl₂
(mes)₂-B-O-Zₙ-C₂H₅
(C₂H₅)₂-Al-O-Al-(C₂H₅)₂
dans lesquelles
iBu représente le radical isobutyle.
mes représente le groupement mésityl (2,4,6 triméthylphényl)

6. Procédé selon l'une de ces revendications 1 à 5, **caractérisé en ce que** le système catalytique comprend un rapport molaire de cocatalyseur par rapport aux moles de Ni compris entre 0.1 et 10.

7. Procédé selon l'une des revendications précédentes, **caractérisé en ce que** le ligand organophosphoré est choisi dans le groupe comprenant les composés organophosphorés monodentate et bidentate.

8. Procédé selon l'une des revendications précédentes, **caractérisé en ce que** les composés organiques à transformer en composés dinitriles sont des composés pentènenitriles.

9. Procédé selon la revendication 8, **caractérisé en ce que** le composé comprenant au moins une fonction nitrile est l'adiponitrile.

## Patentansprüche

1. Verfahren zur Herstellung von Verbindungen mit mindestens einer Nitrilfunktion durch Hydrocyanierung einer organischen Verbindung mit mindestens einer nichtkonjugierten Ungesättigtheit und 2 bis 20 Kohlenstoffatomen durch Reaktion mit Cyanwasserstoff in Gegenwart eines Katalysatorsystems, das einen Komplex von Nickel in der Oxidationsstufe null mit mindestens einem Organophosphorliganden aus der Gruppe umfassend Organophosphite, Organophosphonite, Organophosphinite und Organosphosphine und einen Cokatalysator umfasst, **dadurch gekennzeichnet, dass** es sich bei dem Cokatalysator um eine metallorganische Verbindung der allgemeinen Formel I handelt: 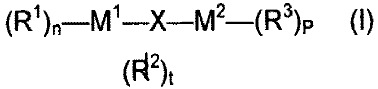 in der:
R¹, R² und R³ gleich oder verschieden sind und für einen verzweigten oder unverzweigten aliphatischen organischen Rest, einen gegebenenfalls substituierten aromatischen oder cycloaliphatischen Rest oder ein Halogenatom stehen,
M¹ und M² gleich oder verschieden sind und für ein Element mit der Wertigkeit m¹ bzw. m² aus der Gruppe umfassend Zink, Bor, Aluminium, Cadmium, Gallium, Indium und Zinn stehen, wobei M¹ und M² nicht gleichzeitig für Bor stehen können,
X für Sauerstoff steht,
n für eine ganze Zahl mit einem Wert von m¹-1 steht,
p für eine ganze Zahl mit einem Wert von m²-1 steht,
t für eine ganze Zahl mit einem Wert von x-2 steht.

2. Verfahren nach Anspruch 1, **dadurch gekennzeichnet, dass** M² für Aluminium oder Zink steht und M¹ für Bor oder Aluminium steht.

3. Verfahren nach Anspruch 1, **dadurch gekennzeichnet, dass** M² für Aluminium oder Zink steht und M¹ für Bor oder Aluminium steht.

4. Verfahren nach Anspruch 1 bis 3, **dadurch gekennzeichnet, dass** die Reste R¹ und R³ für einen Alkylrest mit 1 bis 6 Kohlenstoffatomen, einen gegebenenfalls substituierten Phenylrest oder ein Halogenatom stehen.

5. Verfahren nach einem der vorhergehenden Ansprüche, **dadurch gekennzeichnet, dass** der Cokatalysator aus der Gruppe umfassend die folgenden Verbindungen oder deren Dimere, Trimere oder Tetramere ausgewählt wird:
(C₂H₅)₂-B-O-Al-(C₂H₅)₂
(C₂H₅)₂-B-O-Al-Cl₂
(iBᵤ)₂-Al-O-Al-(iBᵤ)₂
(mes)₂-B-O-Al-(C₂H₅)₂ 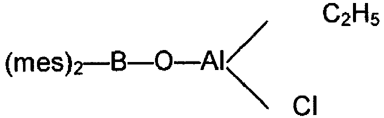
(mes)₂-B-O-Al-Cl₂
(mes)₂-B-O-Zₙ-C2H5
(C₂H₅)₂-Al-O-Al-(C₂H₅)₂
wobei iBu für den Isobutylrest steht,
mes für die Mesitylgruppe (2,4,6-Trimethylphenylgruppe) steht.

6. Verfahren nach einem der Ansprüche 1 bis 5, **dadurch gekennzeichnet, dass** das Katalysatorsystem ein Molverhältnis von Cokatalysator zu Molen Ni zwischen 0,1 und 10 aufweist.

7. Verfahren nach einem der vorhergehenden Ansprüche, **dadurch gekennzeichnet, dass** der Organophosphorligand aus der Gruppe umfassend einzähnige und zweizähnige Organophosphorverbindungen ausgewählt wird.

8. Verfahren nach einem der vorhergehenden Ansprüche, **dadurch gekennzeichnet, dass** es sich bei den in Dinitrilverbindungen umzuwandelnden organischen Verbindungen um Pentennitrilverbindungen handelt.

9. Verfahren nach Anspruch 8, **dadurch gekennzeichnet, dass** es sich bei der Verbindung mit mindestens einer Nitrilfunktion um Adiponitril handelt.

## Claims

1. Process for producing compounds comprising at least one nitrile function by hydrocyanation of an organic compound comprising at least one nonconjugated unsaturation, comprising from 2 to 20 carbon atoms, by reaction with hydrogen cyanide in the presence of a catalytic system comprising a complex of nickel having the oxidation state of zero with at least one organophosphorus ligand chosen from the group comprising organophosphites, organophosphonites, organophosphinites and organosphosphines and a cocatalyst, **characterized in that** the cocatalyst is an organometallic compound corresponding to general formula I: 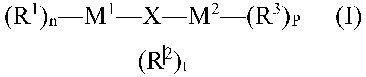 in which:
R¹, R² and R³, which may be identical or different, represent a branched or
unbranched, aliphatic organic radical, a substituted or unsubstituted,
aromatic or cycloaliphatic radical, or a halogen atom,
M¹, M², which may be identical or different, represent an element of valency m¹, m², respectively, chosen from the group comprising zinc, boron, aluminium, cadmium, gallium, indium and tin, it being impossible for M¹ and M² to simultaneously represent boron,
X is an element of valency x chosen from the group comprising oxygen, carbon, nitrogen, silicon, sulphur and phosphorus,
n is an integer equal to m¹-1,
p is an integer equal to m²-1,
t is an integer equal to x-2.

2. Process according to Claim 1, **characterized in that** M² represents aluminium or zinc, and M¹ represents boron or aluminium.

3. Process according to Claim 1, **characterized in that** M² represents aluminium or zinc, M¹ represents boron or aluminium, and X represents oxygen.

4. Process according to Claims 1 to 3, **characterized in that** the radicals R¹ and R³ represent an alkyl radical containing from 1 to 6 carbon atoms, a substituted or unsubstituted phenyl radical, or a halogen atom.

5. Process according to one of the preceding claims, **characterized in that** the cocatalyst is chosen from the group comprising the following compounds or dimers, trimers or tetramers thereof:
(C₂H₅)₂-B-O-Al-(C₂H₅)₂
(C₂H₅)₂-B-O-Al-Cl₂
(iBᵤ)₂-Al-O-Al-(iBᵤ)₂
(mes)₂-B-O-Al-(C₂H₅)₂ 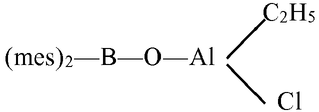
(mes)₂-B-O-Al-Cl₂
(mes)₂-B-O-Zₙ-C₂H₅
(C₂H₅)₂-Al-O-Al-(C₂H₅)₂
in which:
iBu represents the isobutyl radical,
mes represents the mesityl (2,4,6-trimethylphenyl) group.

6. Process according to one of these Claims 1 to 5, **characterized in that** the catalytic system comprises a molar ratio of cocatalyst relative to the moles of Ni of between 0.1 and 10.

7. Process according to one of the preceding claims, **characterized in that** the organophosphorus ligand is chosen from the group comprising monodentate and bidentate organophosphorus compounds.

8. Process according to one of the preceding claims, **characterized in that** the organic compounds to be converted to dinitrile compounds are pentenenitrile compounds.

9. Process according to Claim 8, **characterized in that** the compound comprising at least one nitrile function is adiponitrile, methylglutarolnitrile or succinonitrile.
